Europäisches Patentamt

European Patent Office

Office européen des brevets

(11) Publication number: 0 403 953 A1

# EUROPEAN PATENT APPLICATION

(21) Application number: 90111205.2

(22) Date of filing: 13.06.90

(51) Int. Cl.5: A61B 5/0456

(30) Priority: 23.06.89 JP 161920/89

(43) Date of publication of application:
27.12.90 Bulletin 90/52

(84) Designated Contracting States:
DE FR GB IT

(71) Applicant: FUKUDA DENSHI CO., LTD.
39-4, Hongo 3-chome Bunkyo-ku
Tokyo 113(JP)

(72) Inventor: Maruyama, Mitsuya, c/o Fukuda
Denshi Co.,Ltd.
Hongo enterprise place, 2-35-8 Hongo,
Bunkyo-ku
Tokyo(JP)

(74) Representative: Behn, Klaus, Dipl.-Ing.
Patentanwalt Lindenberg 34
D-8134 Pöcking bei München(DE)

(54) Method and apparatus for detecting QRS complex of ECG.

(57) A method and apparatus for detecting the QRS complex an ECG signal comprises a first threshold computer (7) and a second threshold computer (5) receiving a preprocessed ECG signal (E5).

The first threshold value (T1) is based on a peak value of a QRS complex of the ECG signal (E5) and the second threshold value (T2) is derived from an average value (NA) of the ECG signal (E5). The first threshold value (T1) is compared with the second threshold value (T2), and the larger one of said threshold values (T1 and T2) is selected as a threshold value (T) for detecting the QRS complex. Synchronous signals (SYNC) are delivered in response to each detected QRS complex.

FIG. 3A

EP 0 403 953 A1

# METHOD AND APPARATUS FOR DETECTING QRS COMPLEX OF ECG

## BACKGROUND OF THE INVENTION

### 1. Field of the invention

The present invention relates to a method and an appratus for detecting QRS complex of electrocardiogram (ECG).

More particularly, it relates to a method and an apparatus for detecting QRS complex of ECG, in which QRS complex is detected and a synchronous signal synchronizing with the detected QRS complex is output.

### 2. Description of the Related Art

Generally, a QRS complex is a wave in which Q, R and S waves of ECG are combined, which QRS complex appears just before venticle of the heart contracts, and means that the ventricle is in the pocess of the heart excitation.

Hence, when a synchronous signal synchronizing with the QRS complex is output, it is used as the basis of counting heart beat number.

Before the synchronous signal is output, the QRS complex should be detected previously.

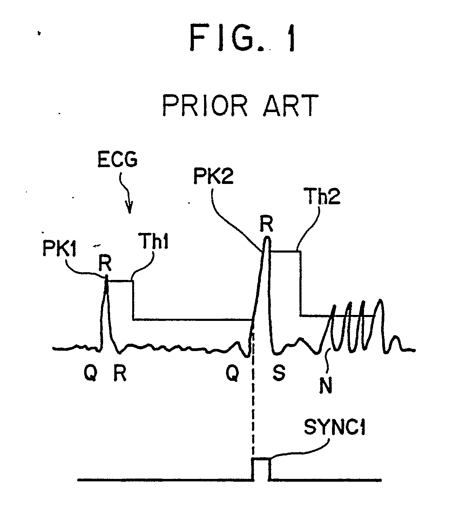

Figure 1 is an explanatory drawing of a prior art, wherein a conventional method for detecting QRS complex of ECG is shown.

Referring to Fig. 1, a threshold valve Th 1 is computed by using a peak valve PK1 of a first QRS complex of ECG, and a wave with the value beyond the threshold value Th1 is detected as a second QRS complex, thereby a synchronus signal SYNC1 synchronizing with the second QRS complex is output.

However, the threshold value computed based on the peak value of QRS complex is fixed.

So, in the prior art as shown in Fig. 1, the influence of noise in cluded in ECG is not considered at all.

Hence, when the nois becomes to grow larger, it is detected accidentally by the fixed threshold value, as the QRS complex.

For example, in Fig. 1, a threshold value Th2 computed with a peak value PK2 of a second QRS complex is used in order to detect a next third QRS complex ( not shown ).

However, when a nois N is included in ECG, the noise N is detected accidentally by using the threshold value Th2, as the third QRS complex.

Accordingly, the problem with the prior art is that the accuracy for detectiug QRS complex is low.

## SUMMARY OF THE INVENTION

An object of the present invention is to improve the accuracy for detecting QRS complex.

The above-mentioned object can be achieved by a method for detecting QRS complex of ECG, comprising the steps of inputting ECG signal E5, setting a first threshold value T1 based on a peak value of QRS complex of said ECG signal E5, setting a second threshold value T2 by presuming a noise level proportional to the sizes of noises included in said ECG signal E5, comparring said first threshold value T1 with said second threshold value T2, deeming larger one of said first and second threshold values T1 and T2 to be a threshold value T for detecting QRS complex, detecting QRS complex by using said threshold value T for detecting QRS complex, and outputting a synchronoussignal SYNC synchronizing with said detected ORS complex, and an apparatus for detecting QRS complex of ECG, comprising a first threshold computing portion 7 which inputs ECGsignal E5, and outputs a first threshold value T1 based on·a peak value of QRS complex of said ECG signal E5, a second threshold computing portion 5 which inputs said ECG signal E5, and outputs a second threshold value T2 by presuming a noise level proportional to the sizes of noises included in said ECG signal E5, a threshold comparring portion 6 which inputs said first and second threshold value T1 and T2, compares the sizes of them, and outputs a larger one of them as a threshold value T for detecting QRS complex, and a synchronous signal outputting portion 8 which inputs said threshold value T for detecting QRS complex and said ECG signal E5, detects QRS complex of said ECG signal E5 by using said threshold value T for detecting QRS, and outputs synchronous signal SYNC synchronizing with said detected QRS complex.

## BRIEF DESCRIPTION OF THE DRAWINGS

Further features and advantages of the present invention will be apparent from the ensuring description with reference to the accompanying drawings, wherein:

Fig. 1 is an explanatory drawing of the prior art;

Fig. 2 is a drawing of a method in accordance with the present invention;

Fig. 3A is a drawing of an embodiment of an apparatus in accordance with the present invention;

Fig. 3B is an explanatory drawing for operating an apparatus of Fig. 3A; and

Fig. 3C is another explanatory drawing for operating an apparatus of Fig. 3A.

## DESCRIPTION OF THE PREFERRED EMBODIMENTS

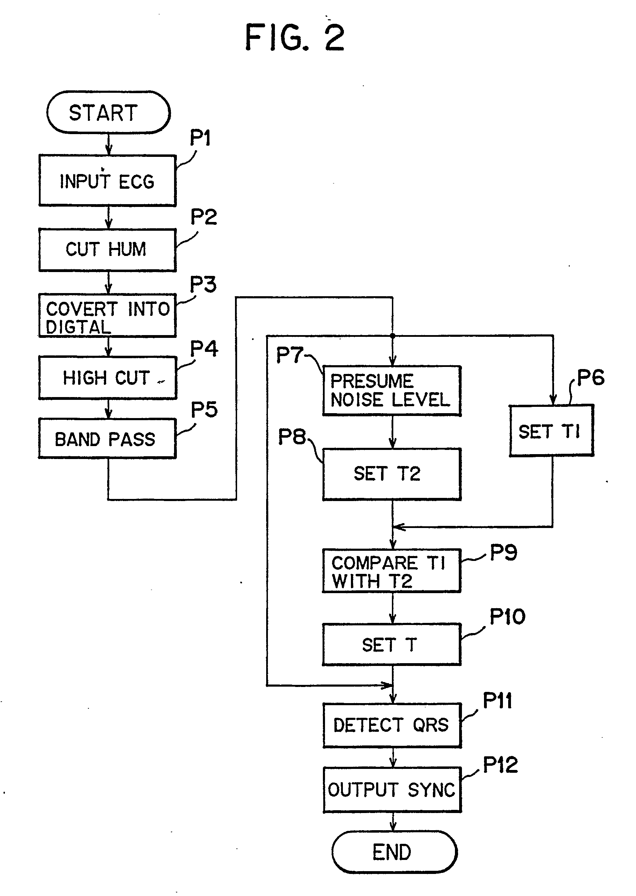

Figure 2 is a drawing of an embodiment of a method in accordance with the present invention

First, electrocardiogram (ECG) is input from the living body of a patient, in a step P1.

In a step P2, hum included in ECG is cut.

ECG, of which hum is cut, is converted into digtal from analog, in a step P3.

High freguency of ECG is cut in a step P4, and the power of ECG is passed in a certain band width in a step P5.

Thereafter, ECG is treated in steps 6 and 7.

In the step 6, a peak value of ECG is detected, and a first threshold value T1 is setted on the base of the peak value.

In the step 7, a level of noise included in ECG is presumed, and a first thereshold value T2 is setted on the base of the presumed noise level, in a step P8.

The sizes of the first and second threshold values T1 and T2 are compared in a step P9, and a larger one of the above threshold values T1 and T2 is setted as a threshold value T for detecting QRS complex, in a step P10.

In a step P11, QRS complex is detected on the base of the QRS complex detecting threshold value T and ECG treated in the step 5.

In a step 12, a synchronous signal synchronizing with the detected QRS complex is output.

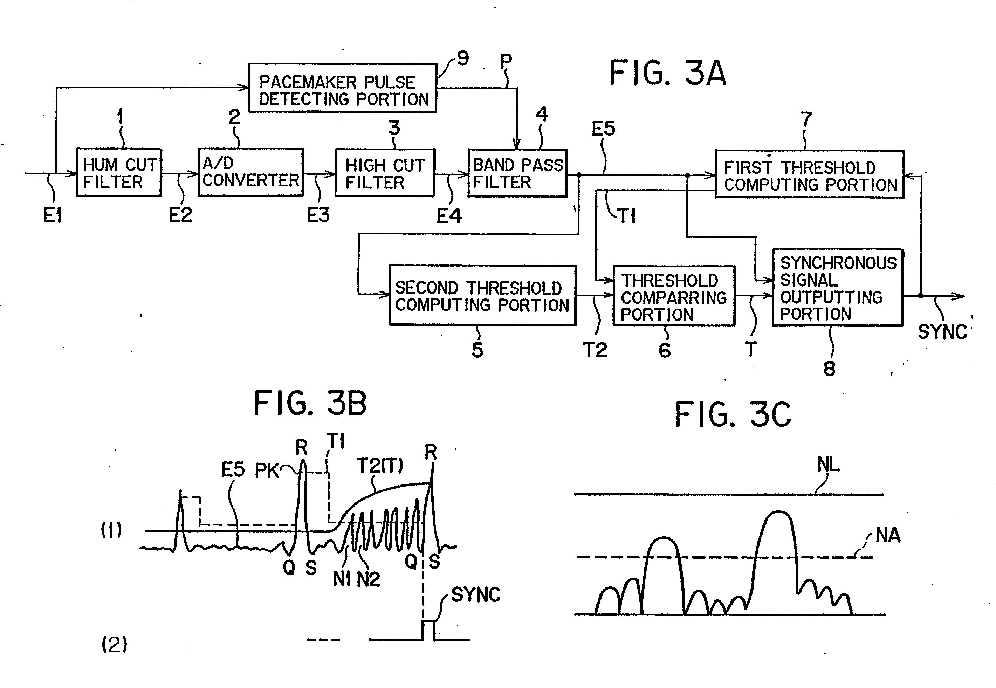

Figure 3A is a drawing of an embodiment of an apparatus in accordance with the present invention, wherein reference numeral 1 shows a hum cut filter, 2 A/D converter, 3 high cut filter, 4 a band pass filter, 5 a second threshold computing portion, 6 a threshold comparring portion, 7 a first threshold computing portion, 8 a synchronous signal outputting portion, and 9 a pacemaker pulse detecting portion.

Fig. 3B is an explanatory drawing for operating an apparatus of Fig. 3A, wherein waveformes of signals output are shown.

Fig. 3C is another explanatory drawing for operating an apparatus of Fig. 3A, wherein an way for presuming a noise level is shown.

Electrocardiogram (ECG) signal E1 led from the living body of a patient is input into the hum cut filter 1 and the pacemaker pulse detecting portion 9.

Hum included in the ECG signal E1 is removed in the hum cut filter 1, and ECG signal E2 without hum is output.

The ECG signal E2 is input into the A/D converter 2, therein it is converted from analogue signal into digital signal.

Consequently, digtal ECG signal E3 is output from the A/D converter 2.

The digital ECG signal E3 is input into the high cut filter 3, wherein high frequency of it is cut.

Accordingly, high cut ECG signal E4 is output from the high cut filter 3, and it is input into the band pass filter 4.

In the band pass filter 4, the power of the ECG signal E4 is passed in only a certain band width, whereby ECG signal E5 as shown in Fig. 3B (1) is output.

Since pulse P output from the pacemaker pulse detecting portion 9 is input into the band pass filter 4, wherein the pulse P is removed.

Thereby, the pulse P of a pacemaker is not included in the ECG signal E5.

The ECG signal E5 is input into the first and second threshold computing portions 5 and 7, and the synchronous signal outputting portion.

In the second threshold computing portion 5, a level of noise induded in the ECG signal E5 is presumed as shown in Fig. 3C.

That is to say, an average peak valve NA of the ECG signal E5 is computed by means of deeming the ECG signal E5 to be a half wave rectified sign wave.

However, if the average peak value NA be considered the noise level, when a large noise is included in the ECG signal E5, it is afraid that the large noise is detected accidentally as the QRS complex.

Hence, in order that there is sufficient margin to detect QRS complex, a level NL twice the average peak value NA is presumed to be the noise level.

A formula for expressing the level NL is as follows.

$$NL = 2NA = 4 Y_n = 4 \{Y_{n-1} + 1 / K (X_n - Y_{n-1})\}$$

In the above formula, Xn shows the value of an input signal at n time into the second threshold computing portion 5, and Yn shows the value of an output signal at n time from the same portion 5.

Based on noïses N1, N2 . . . included in the ECG signal E5 (see Fig. 3B(1)), the noise level NL is presumed according to the above way as shown in Fig 3C, and it is setted as the second threshold value T2 (see Fig. 3B(1)).

As apparenly from Fig. 3B, the second threshold value T2 is porportional to the sizes of the noies N1, N2 . . . and it is very larger than them.

The second threshold value T2 is input into the threshold comparring portion 6.

On the other hand, in the first threshold computing por tion 7, a first threshold value T1 is computed based on a peak value PK (see Fig.3B (1)) of the QRS complex of the ECG signal E5, which first threshold value T1 is input into the threshold comparring portion 6.

Accordingly, in the threshold comparring portion 6, the sizes of the first threshold value T1 and the second threshold value T2 are compared.

Since the second threshold value T2 is larger than the first threshold value T1 at the part of the noises N1, N2 . . ., it is considered a threshold value T for detecting QRS complex, which threshold value T is input into the synchronous signal outputting portion 8.

In the synchronous outputting portion 8, wherein the ECG signal E5 has been already input, next QRS complex is detected by using the threshold value T for detecting QRS complex, thereafter a synchronous signal SYNC synchronizing with the detected QRS complex is output, as shown in Fig. 3B (2).

According to the present invention, the second threshold value T2 is setted by presuming a nois level proportional to the sizes of noise included in the ECG signal E5, which second threshold value T2 is compared with the first threshold value T1 computed based on the peak value PK of the ECG signal E5.

Thereby, a larger one of the first and second threshold values T1 and T2 is deemed to be the threshold value T for detecting QRS complex, with which value T next QRS complex can be detected.

Consequently, since the influence of nois is considered sufficiently by the present invention, it does not happen that large nois is detected accidentally as QRS complex like the prior art does.

Hence, the accuracy for detecting QRS complex has been improved remarkably.

## Claims

1. A method for detecting QRS complex of ECG, comprising;
the steps of inputting ECG signal E5,
setting a first threshold value T1 based on a peak value of QRS complex of said ECG signal E5,
setting a second threshold value T2 by presuming a noise level proportional to the sizes of noises included in said ECG signal E5,
comparring said first threshold value T1 with said second threshold value T2,
deeming larger one of said first and second threshold values T1 and T2 to be a threshold value T for detecting QRS complex,
detecting QRS complex by using said threshold value T for detecting QRS complex, and
outputting a synchronoussignal SYNC synchronizing with said detected QRS complex.

2. A method for detecting QRS complex of ECG according to claim 1, wherein a value twice an average peak value NA of said ECG signal E5 is presumed to be a noise level NL.

3. An apparatus for detecting QRS complex of ECG, comprising; a first threshold computing portion 7 which inputs ECG signal E5, and outputs a first threshold value T1 based on a peak value of QRS complex of said ECG signal E5,
a second threshold computing portion 5 which inputs said ECG signal E5, and outputs a second threshold value T2 by presuming a noise level proportional to the sizes of noises included in said ECG signal E5,
a threshold comparring portion 6 which inputs said first and second threshold value T1 and T2, compares the sizes of th30 em, and outputs a larger one of them as a threshold value T for detecting QRS complex, and
a synchronous signal outputting portion 8 which inputs said threshold value T for detecting QRS complex and said ECG signal E5, detects QRS complex of said ECG signal E5 by using said threshold value T for detecting QRS, and outputs synchronous signal SYNC synchronizing with said detected QRS complex.

# FIG. 1

## PRIOR ART

# FIG. 2

```
        START
          │ P1
     ┌──────────┐
     │ INPUT ECG │
     └──────────┘
          │ P2
     ┌──────────┐
     │ CUT HUM  │
     └──────────┘
          │ P3
     ┌──────────────┐
     │ COVERT INTO  │
     │ DIGTAL       │
     └──────────────┘
          │ P4
     ┌──────────┐
     │ HIGH CUT │
     └──────────┘
          │ P5
     ┌──────────┐
     │ BAND PASS│
     └──────────┘
```

P7 ┌──────────────┐            ┌──────────┐ P6
   │ PRESUME      │            │ SET TI   │
   │ NOISE LEVEL  │            └──────────┘
   └──────────────┘

P8 ┌──────────┐
   │ SET T2   │
   └──────────┘

   ┌──────────────┐ P9
   │ COMPARE TI   │
   │ WITH T2      │
   └──────────────┘

   ┌──────────┐ P10
   │ SET T    │
   └──────────┘

   ┌──────────┐ P11
   │ DETECT QRS│
   └──────────┘

   ┌──────────────┐ P12
   │ OUTPUT SYNC  │
   └──────────────┘

        END

## FIG. 3A

E1 → **HUM CUT FILTER** (1)

E2 → **A/D CONVERTER** (2)

E3 → **HIGH CUT FILTER** (3)

**PACEMAKER PULSE DETECTING PORTION** (9)

P

E4 → **BAND PASS FILTER** (4)

E5

**SECOND THRESHOLD COMPUTING PORTION** (5)

T1

T2

**THRESHOLD COMPARRING PORTION** (6)

T

**FIRST THRESHOLD COMPUTING PORTION** (7)

**SYNCHRONOUS SIGNAL OUTPUTTING PORTION** (8)

→ SYNC

## FIG. 3B

(1) E5 PK R R T1 T2(T) R Q S Q S N1 N2

(2) SYNC

## FIG. 3C

NL

NA

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | US-A-4769760 (M.W. KROLL ET AL.) <br> * column 6, line 16 - column 7, line 6 * <br> * column 8, line 36 - column 9, line 68; figures * <br> --- | 1, 3 | A61B5/0456 |
| Y | US-A-4237903 (G.H. HOFMANN) <br> * column 3, line 17 - column 4, line 25 * <br> * column 4, line 64 - column 5, line 4; figures * <br> --- | 1, 3 | |
| A | US-A-3599033 (R.L. STETTINER ET AL.) <br> * column 3, lines 19 - 33; figure 1 * <br> --- | 1, 3 | |
| A | US-A-3821948 (E. KING) <br> * column 4, lines 20 - 45 * <br> * column 5, lines 12 - 32 * <br> * column 6, lines 7 - 48; figures * <br> --- | 1, 3 | |
| A | DE-A-3533912 (W. SCHMID) <br> * column 6, line 40 - column 7, line 20; figures * <br> ----- | 1, 3 | |
| | | | **TECHNICAL FIELDS SEARCHED (Int. Cl.5 )** <br><br> A61B |

The present search report has been drawn up for all claims

| Place of search | Date of completion of the search | Examiner |
|---|---|---|
| THE HAGUE | 18 SEPTEMBER 1990 | RIEB K.D. |

EPO FORM 1503 03.82 (P0401)